# EUROPEAN PATENT SPECIFICATION

(11) **EP 0 614 463 B1**
(45) Date of publication and mention of the grant of the patent: **12.02.2003**
(21) Application number: 93900609.4
(22) Date of filing: 23.11.1992
(51) Int. Cl.: C07J 5/00, A61K 31/56, C07J 3/00, C07J 51/00, C07J 11/00, C07J 9/00, C07J 41/00

(54) **ANGIOSTATIC STEROIDS**
ANGIOSTATISCHE STEROIDE
STERO DES ANGIOSTATIQUES

(30) Priority: 22.11.1991 US 796169; 02.06.1992 US 892448; 08.09.1992 US 941485
(43) Date of publication of application: 14.09.1994
(60) Divisional application: 02009991.7
(73) Proprietor: ALCON LABORATORIES, INC., Ft. Worth, TX 76134 (US)
(72) Inventor: CLARK, Abbot, F., Arlington, TX 76017 (US); CONROW, Raymond, E., Fort Worth, TX 76133 (US)
(74) Representative: Keller, Günter, Dr.
(86) International application number: US9210133
(87) International publication number: WO93010141

(56) References cited:
- EP-A- 0 250 088
- EP-A- 0 280 797
- EP-A- 0 371 617
- WO-A-86/02554
- WO-A-87/02672
- WO-A-89/01482
- WO-A-90/15816
- WO-A-91/03245
- WO-A-91/19731
- GB-A- 790 452
- US-A- 3 658 856
- US-A- 4 041 055
- US-A- 4 342 702
- US-A- 4 357 279
- US-A- 4 600 538
- US-A- 5 036 048
- INVESTIGATIVE OPTHALMOLOGY AND VISUAL SCIENCE vol. 28, no. 5, May 1987, NEW YORK US pages 901 - 903 A. L. SOUTHREN ET AL 'Intraocular Hypotensive Effect of a Topically Administered Cortisol Metabolite: 3-alpha,5-beta-Tetrahydrocortisol'
- DRUGS OF THE FUTURE vol. 9, no. 10, October 1984, pages 755 - 757 P. G. ADAIKAN ET AL 'RU-486'
- CHEMICAL ABSTRACTS, vol. 90, no. 25, 18 June 1979, Columbus, Ohio, US; abstract no. 198072, A. GIERKOWS ET AL 'Effect of Steroid Hormones: Estrogens and Progesterone, as well as Chorionic Gonadotrophin and LH-releasing Factor on the Structure of the Filtration Angle and Intraocular Tension in Rabbits' page 89 ;column 2 ;
- CHEMICAL ABSTRACTS, vol. 110, no. 5, 30 January 1989, Columbus, Ohio, US; abstract no. 33913, M. PRESTA 'Sex Hormones Modulate the Synthesis of Basic Fibroblast Growth Factor in Human Endometrial Adenocarcinoma Cells: Implications for the Neovascularisation of Normal and Neoplastic Endometrium' page 69 ;column 2 ;
- SCIENCE vol. 230, no. 4732, 20 December 1985, LANCASTER, PA US pages 1375 - 1378 R. CRUM ET AL 'A New Class of Steroids Inhibits Angiogenesis in the Presence of Heparin or a Heparin Fragment'
- CHEMICAL ABSTRACTS, vol. 117, no. 6, 10 August 1992, Columbus, Ohio, US; abstract no. 56002, K. TRIER ET AL 'The Use of Estrogens in the Preparation of Formulations for Topical Treatment of High Pressure in the Eyes' page 480 ;column 1 ;
- CHEMICAL ABSTRACTS, vol. 102, Columbus, Ohio, US; abstract no. 219200,
- CHEMICAL ABSTRACTS, vol. 95, Columbus, Ohio, US; abstract no. 74213,
- CHEMICAL ABSTRACTS, vol. 95, Columbus, Ohio, US; abstract no. 54862,
- CHEMICAL ABSTRACTS, vol. 105, Columbus, Ohio, US; abstract no. 146246,
- CHEMICAL ABSTRACTS, vol. 90, Columbus, Ohio, US; abstract no. 115651,
- CHEMICAL ABSTRACTS, vol. 98, Columbus, Ohio, US; abstract no. 194208,
- CHEMICAL ABSTRACTS, vol. 107, Columbus, Ohio, US; abstract no. 190525,
- CHEMICAL ABSTRACTS, vol. 110, Columbus, Ohio, US; abstract no. 88301,
- CHEMICAL ABSTRACTS, vol. 112, Columbus, Ohio, US; abstract no. 70199,
- CHEMICAL ABSTRACTS, vol. 102, Columbus, Ohio, US; abstract no. 100761,
- CHEMICAL ABSTRACTS, vol. 76, Columbus, Ohio, US; abstract no. 54648,
- CHEMICAL ABSTRACTS, vol. 83, Columbus, Ohio, US; abstract no. 1218,
- CHEMICAL ABSTRACTS, vol. 86, Columbus, Ohio, US; abstract no. 133829,
- CHEMICAL ABSTRACTS, vol. 101, Columbus, Ohio, US; abstract no. 84154,
- PROSTAGLANDINS vol. 11, no. 3, 1976, pages 591 - 594
- CHEMICAL ABSTRACTS, vol. 94, Columbus, Ohio, US; abstract no. 132880,
- CHEMICAL ABSTRACTS, vol. 68, Columbus, Ohio, US; abstract no. 19235,
- CHEMICAL ABSTRACTS, vol. 79, Columbus, Ohio, US; abstract no. 100867,
- CHEMICAL ABSTRACTS, vol. 92, Columbus, Ohio, US; abstract no. 34473,
- CHEMICAL ABSTRACTS, vol. 91, Columbus, Ohio, US; abstract no. 102746,
- CHEMICAL AND PHARMACEUTICAL BULLETIN vol. 33, no. 5, 1985, pages 1889 - 1898
- DATABASE WPI Week 199149, Derwent Publications Ltd., London, GB; Class B01, AN 1991-356589 & JP 03 236 324 A (TAKEDA CHEM. IND. LTD) 22 October 1991
- CANCER CHEMOTHER. vol. 51, no. 4, 1967, pages 225 - 228

## Description

### Background of the Invention

### Field of the Invention

This invention relates to the use of an angiostatic steroid for the preparation of a medicament for preventing and treating neovascularization.

### Description of Related Art

Steroids functioning to inhibit angiogenesis in the presence of heparin or specific heparin fragments are disclosed in Crum, et al., *A New Class of Steroids Inhibits Angiogenesis in the Presence of Heparin or a Heparin Fragment,* Science, Vol.230, pp.1375-1378 (December 20, 1985). The authors refer to such steroids as "angiostatic" steroids. Included within the new class of steroids found to be angiostatic are the dihydro and tetrahydro metabolites of cortisol and cortexolone. In a follow-up study directed to testing a hypothesis as to the mechanism by which the steroids inhibit angiogenesis, it was shown that heparin/angiostatic steroid compositions cause dissolution of the basement membrane scaffolding to which anchorage dependent endothelia are attached resulting in capillary involution; see, Ingber, et al., *A Possible Mechanism for Inhibition of Angiogenesis by Angiostatic Steroids: Induction of Capillary Basement Membrane Dissolution,* Endocrinology Vol. 119, pp.1768-1775 1986).

A group of tetrahydro steroids useful in inhibiting angiogenesis is disclosed in International Patent Application No WO-87/02672, Aristoff, et al., (The Upjohn Company). The compounds are disclosed for use in treating head trauma, spinal trauma, septic or traumatic shock, stroke and hemorrhage shock. In addition, the patent application discusses the utility of these compounds in embryo implantation and in the treatment of cancer, arthritis and arteriosclerosis. Some of the steroids disclosed in Aristoff et al. are disclosed in U.S. Patent No. 4,771,042 in combination with heparin or a heparin fragment for inhibiting angiogenesis in a warm blooded animal.

Compositions of hydrocortisone, "tetrahydrocortisol-S," and U-72,745G, each in combination with a beta cyclodextrin, have been shown to inhibit corneal neovascularization: Li, et al., *Angiostatic Steroids Potentiated by Sulphated Cyclodextrin Inhibit Corneal Neovascularization,* Investigative Ophthalmology and Visual Science, Vol. 32, No. 11, pp. 2898-2905 (October, 1991). The steroids alone reduce neovascularization somewhat but are not effective alone in effecting regression of neovascularization.

Tetrahydrocortisol (THF) has been disclosed for its use in lowering the intraocular pressure (IOP) of rabbits made hypertensive with dexamethasone alone, or with dexamethasone/5-beta-dihydrocortisol; see Southren, et al., *Intraocular Hypotensive Effect of a Topically Applied Cortisol Metabolite: 3-alpha, 5-beta-tetrahydrocortisol*, Investigative Ophthalmology and Visual Science, Vol.28 (May, 1987). The authors suggest THF may be useful as an antiglaucoma agent. In U.S. Patent No. 4,863,912, issued to Southren et al. on September 5, 1989, pharmaceutical compositions containing THF and a method for using these compositions to control intraocular pressure are disclosed. THF has been disclosed as an angiostatic steroid in Folkman, et al., *Angiostatic Steroids,* Ann. Surg., Vol.206, No.3 (1987) wherein it is suggested angiostatic steroids may have potential use for diseases dominated by abnormal neovascularization, including diabetic retinopathy, neovascular glaucoma and retrolental fibroplasia.

### Summary of the Invention

This invention is directed to the use of an angiostatic steroid for the preparation of a medicament for inhibiting neovascularization. The compositions containing the steroid can be used for treatment of angiogenesis dependent diseases, for example: head trauma, spinal trauma, septic or traumatic shock, stroke, hemorrhagic shock, cancer, arthritis, arteriosclerosis, angiofibroma, arteriovenous malformations, corneal graft neovascularization, delayed wound healing, diabetic retinopathy, granulations, burns, hemangioma, hemophilic joints, hypertrophic scars, neovascular glaucoma, nonunion fractures, Osler-Weber Syndrome, psoriasis, pyogenic granuloma, retrolental fibroplasia, pterigium, scleroderma, trachoma, vascular adhesions, and solid tumor growth. In particular, the angiostatic steroids and compositions thereof are useful for controlling ocular neovascularization.

### Brief Description of the Drawing

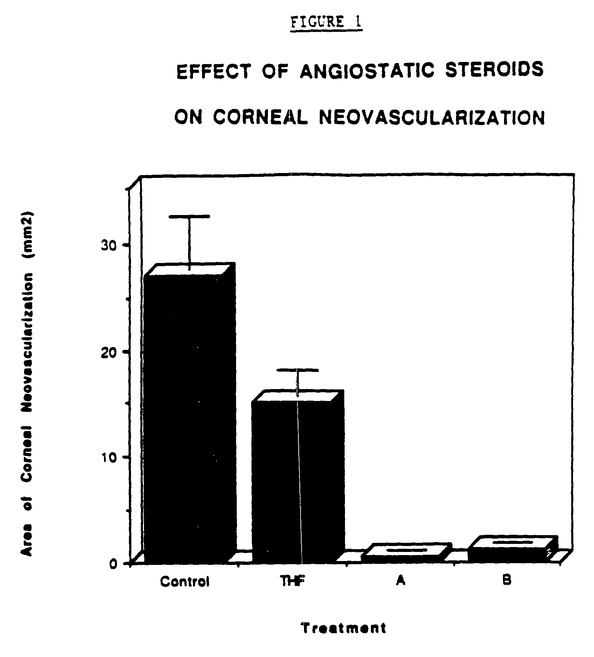

Figure 1 compares the ability of angiostatic steriods to inhibit neovascularization in the rabbit cornea.

### Detailed Description of Preferred Embodiments

The development of blood vessels for the purpose of sustaining viable tissue is known as angiogenesis or neovascularization. Agents which inhibit neovascularization are known by a variety of terms such as angiostatic, angiolytic or angiotropic agents. For purposes of this specification, the term "angiostatic agent" means compounds which can be used to control prevent, or inhibit angiogenesis.

The angiostatic agent to be used according to the present invention is a steroid or steroid metabolite. For purposes herein, the term "angiostatic steroid" means a steroid and steroid metabolites which inhibit angiogenesis.

There is currently no effective method for controlling the neovascularization in angiogenesis-dependent diseases. In particular, ocular neovascularization has not been successfully treated in the past. Neovascularization of tissues in the front of the eye (i.e. the cornea, iris, and the trabecular meshwork) and other conditions, including conditions in the back of the eye, for example, retinal, subretinal, macular, and optical nerve head neovascularization, can be prevented and treated by administration of the steroid of this invention. The angiostatic steroid defined in the present invention is useful in preventing and treating neovascularization, including providing for the regression of neovascularization.

The angiostatic steroid used according to the present invention has the following formula: 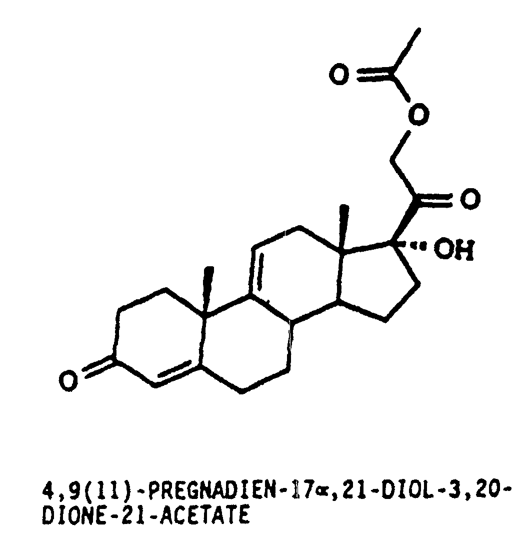 The above structure includes all pharmaceutically acceptable salts of the angiostatic steroid.

4,9(11)-Pregnadien-17α,21-diol-3,20-dione-21-acetate is a compound for preventing and treating neovascularization.

The angiostatic steroid which can be used according to the present invention is useful in inhibiting neovascularization and can be used in treating the neovascularization associated with: head trauma, spinal trauma, systemic or traumatic shock, stroke, hemorrhagic shock, cancer, arthritis, arteriosclerosis, angiofibroma, arteriovenous malformations, corneal graft neovascularization, delayed wound healing, diabetic retinopathy, granulations, burns, hemangioma, hemophilic joints, hypertrophic scars, neovascular glaucoma, nonunion fractures, Osler-Weber Syndrome, psoriasis, pyogenic granuloma, retrolental fibroplasia, pterigium, scleroderma, trachoma, vascular adhesions, and solid tumor growth.

In particular, the angiostatic steroid is useful in preventing and treating any ocular neovascularization, including, but not limited to: retinal diseases (diabetic retinopathy, chronic glaucoma, retinal detachment, sickle cell retinopathy, senile macular degeneration due to subretinal neovascularization); rubeosis iritis; inflammatory diseases; chronic uveitis; neoplasms (retinoblastoma, pseudoglioma); Fuchs' heterochromic iridocyclitis; neovascular glaucoma; corneal neovascularization (inflammatory, transplantation, developmental hypoplasia of the iris); neovascularization resulting following a combined vitrectomy and lensectomy; vascular diseases (retinal ischemia, choroidal vascular insufficiency, choroidal thrombosis, carotid artery ischemia); pterigium; neovascularization of the optic nerve; and neovascularization due to penetration of the eye or contusive ocular injury.

The initiation of new blood vessel formation may arise quite differently in various tissues or as a result of different diseases. Many substances have been found to induce neovascularization, see, Folkman, et al., *Angiogenic Factors,* Science, Volume 235, pp. 442-447 (1987). However, it is believed, that once initiated, the process of neovascularization is similar in all tissues regardless of the associated disease, Furcht, *Critical Factors Controlling Angiogenesis: Cell Products, Cell Matrix, and Growth Factors,* Laboratory Investigation, Volume 55, No. 5, pp. 505-509 (1986).

There are a variety of theories regarding the mechanism of action of angiostatic steroids. For example, angiostatic steroid induced inhibition of neovascularization may occur due to, dissolution of the capillary basement membrane, Ingber, et al., *Supra;* inhibition of vascular endothelial cell proliferation, Cariou, et al., *Inhibition of Human Endothelial Cell Proliferation by Heparin and Steroids,* Cell Biology International Reports, Vol. 12, No. 12, pp. 1037-1047 (December, 1988); effect on vascular endothelial cell laminin expression, Tokida, et al., *Production of Two Variant Laminin Forms by Endothelial Cells and Shift of Their Relative Levels by Angiostatic Steroids,* The Journal of Biological Chemistry, Vol. 264, No. 30, pp. 18123-18129 (October 25, 1990); inhibition of vascular cell collagen synthesis, Maragoudakis, et al., *Antiangiogenic Action of Heparin Plus Cortisone is Associated with Decreased Collagenous Protein Synthesis in the Chick Chorioallantoic Membrane System,* The Journal of Pharmacology and Experimental Therapeutics, Vol. 251, No. 2, pp. 679-682 (1989); and inhibition of vascular endothelial cell plasminogen activator activity, Ashino-Fuse, et al., *Medroxyprogesterone Acetate, An Anti-Cancer and Anti-Angiogenic Steroid, Inhibits the Plasminogen Activator in Bovine Endothelial Cells,* Int. J. Cancer, 44, pp. 859-864 (1989).

There are many theories associated with the cause of neovascularization, and there may be different inducers depending on the disease or surgery involved, BenEzra, *Neovasculogenic Ability of Prostaglandins, Growth Factors, and Synthetic Chemoattractants,* American Journal of Ophthalmology, Volume 86, No. 4, pp. 455-461, (October, 1978). Regardless of the cause or the associated disease or surgery, it is believed that angiostatic agents work by inhibiting one or more steps in the process of neovascularization. Therefore, the angiostatic steroid as defined in the present invention is useful in the treatment and prevention of neovascularization associated with a variety of diseases and surgical complications.

The angiostatic steroid used according to the present invention may be incorporated in various formulations for delivery. The type of formulation (topical or systemic) will depend on the site of disease and its severity. For administration to the eye, topical formulations can be used and can include ophthalmologically acceptable preservatives, surfactants, viscosity enhancers, buffers, sodium chloride, and water to form aqueous sterile ophthalmic solutions and suspensions. In order to prepare sterile ophthalmic ointment formulations, an angiostatic steroid is combined with a preservative in an appropriate vehicle, such as mineral oil, liquid lanolin, or white petrolatum. Sterile ophthalmic gel formulations comprising the angiostatic steroid defined in the present invention can be prepared by suspending the angiostatic steroid in a hydrophilic base prepared from a combination of, for example, Carbopol® (a carboxy vinyl polymer available from the BF Goodrich Company) according to published formulations for analogous ophthalmic preparations. Preservatives and antimicrobial agents may also be incorporated in such gel formulations. Systemic formulations for treating ocular neovascularization can also be used, for example, orally ingested tablets and formulations for intraocular and periocular injection.

The specific type of formulation selected will depend on various factors, such as the angiostatic steroid or its salt being used, the dosage frequency, and the location of the neovascularization being treated. Topical ophthalmic aqueous solutions, suspensions, ointments, and gels are the preferred dosage forms for the treatment of neovascularization in the front of the eye (the cornea, iris, trabecular meshwork); or neovascularization of the back of the eye if the angiostatic agent can be formulated such that it can be delivered topically and the agent is able to penetrate the tissues in the front of the eye. The angiostatic steroid will normally be contained in these formulations in an amount from about 0.01 to about 10.0 weight/percent. Preferable concentrations range from about 0.1 to about 5.0 weight/percent. Thus, for topical administration, these formulations are delivered to the surface of the eye one to six times a day, depending on the routine discretion of the skilled clinician. Systemic administration, for example, in the form of tablets is useful for the treatment of neovascularization particularly of the back of the eye, for example, the retina. Tablets containing 10-1000 mg of angiostatic agent can be taken 2-3 times per day depending on the discretion of the skilled clinician.

The following examples illustrate formulations of the compound to be used according to the present invention, but are in no way limiting.

The reference examples are for information purposes.

### Example 1

The topical compositions are useful for controlling ocular hypertension or controlling ocular neovascularization.

| Component | wt .% |
|---|---|
| Angiostatic Steroid | 0.005-5.0 |
| | |
| Tyloxapol | 0.01-0.05 |
| HPMC | 0.5 |
| Benzalkonium Chloride | 0.01 |
| Sodium Chloride | 0.8 |
| Edetate Disodium | 0.01 |
| NaOH/HCl | q.s. pH 7.4 |
| Purified Water | q.s. 100 mL |

### Reference Example 2

The composition is useful for controlling ocular hypertension.

| Component | wt.% |
|---|---|
| 21-Nor-5β-pregnan-3α,17α,20-triol | 1.0 |
| | |
| Tyloxapol | 0.01-0.05 |
| HPMC | 0.5 |
| Benzalkonium Chloride | 0.01 |
| Sodium Chloride | 0.8 |
| Edetate Disodium | 0.01 |
| NaOH/HCl | q.s. pH 7.4 |
| Purified Water | q.s. 100 mL |

The above formulation is prepared by first placing a portion of the purified water into a beaker and heating to 90°C. The hydroxypropylmethylcellulose (HPMC) is then added to the heated water and mixed by means of vigorous vortex stirring until all of the HPMC is dispersed. The resulting mixture is then allowed to cool while undergoing mixing in order to hydrate the HPMC. The resulting solution is then sterilized by means of autoclaving in a vessel having a liquid inlet and a hydrophobic, sterile air vent filter.

The sodium chloride and the edetate disodium are then added to a second portion of the purified water and dissolved. The benzalkonium chloride is then added to the solution, and the pH of the solution is adjusted to 7.4 with 0.1M NaOH/HCl. The solution is then sterilized by means of filtration.

21-Nor-5β-pregnan-3α,17α,20-triol is sterilized by either dry heat or ethylene oxide. If ethylene oxide sterilization is selected, aeration for at least 72 hours at 50°C. is necessary. The sterilized steroid is weighed aseptically and placed into a pressurized ballmill container. The tyloxapol, in sterilized aqueous solution form, is then added to the ballmill container. Sterilized glass balls are then added to the container and the contents of the container are milled aseptically at 225 rpm for 16 hours, or until all particles are in the range of approximately 5 microns.

Under aseptic conditions, the micronized drug suspension formed by means of the preceding step is then poured into the HPMC solution with mixing. The ballmill container and balls contained therein are then rinsed with a portion of the solution containing the sodium chloride, the edetate disodium and benzalkonium chloride. The rinse is then added aseptically to the HPMC solution. The final volume of the solution is then adjusted with purified water and, if necessary, the pH of the solution is adjusted to pH 7.4 with NaOH/HCl.

### Reference Example 3

The following formulation is representative of an antiinflammatory composition.

| Component | wt.% |
|---|---|
| 4,9(11)Pregnadien-17α,21-diol-3,20-dione-21-acetate | 1.0 |
| | |
| Dexamethasone | 0.1 |
| Tyloxapol | 0.01 to 0.05 |
| HPMC | 0.5 |
| Benzalkonium Chloride | 0.01 |
| Sodium Chloride | 0.8 |
| Edetate Disodium | 0.01 |
| NaOH/HCl | q.s. pH 7.4 |
| Purified Water | q.s. 100 mL |

The above formulation is prepared in the same manner set forth in Example 2, sterilizing and adding the dexamethasone to the steroid before placing both into a pressurized ballmill container.

### Reference Example 4

The following formulation is another example of an antiinflammatory composition.

| | wt.% |
|---|---|
| Tetrahydrocortisol | 1.0 |
| Prednisolone Acetate | 1.0 |
| Tyloxapol | 0.01 to 0.05 |
| HPMC | 0.5 |
| Benzalkonium Chloride | 0.01 |
| Sodium Chloride | 0.8 |
| Edetate Disodium | 0.01 |
| NaOH/HCl | q.s. pH 7.4 |
| Purified Water | q.s. 100 mls |

The above formulation is prepared in the same manner set forth in Example 2, sterilizing and adding the prednisolone acetate to the steroid before placing both into a pressurized ballmill container.

The following formulations are representative of compositions used for the treatment of angiogenesis dependent diseases.

### Example 5

| FORMULATION FOR ORAL ADMINISTRATION |
|---|
| Tablet: |
| 10-1000 mg of angiostatic steroid with inactive ingredients such as starch, lactose and magnesium stearate can be formulated according to procedures known to those skilled in the art of tablet formulation. |

### Example 6

| FORMULATION FOR STERILE INTRAOCULAR INJECTION | |
|---|---|
| each mL contains: | |
| Angiostatic Steroid | 10-100 mg |
| Sodium Chloride | 7.14 mg |
| Potassium Chloride | 0.38 mg |
| Calcium chloride dihydrate | 0.154 mg |
| Magnesium chloride hexahydrate | 0.2 mg |
| Dried sodium phosphate | 0.42 mg |
| Sodium bicarbonate | 2.1 mg |
| Dextrose | 0.92 mg |
| Hydrochloric acid or sodium hydroxide to adjust pH to approximately 7.2 | |
| Water for injection | |

### Reference Example 7

| FORMULATION FOR TOPICAL OCULAR SOLUTION | |
|---|---|
| 21-Nor-5α-pregnan-3α,17α-20-triol-3-phosphate | 1.0% |
| Benzalkonium chloride | 0.01% |
| HPMC | 0.5% |
| Sodium chloride | 0.8% |
| Sodium phosphate | 0.28% |
| Edetate disodium | 0.01% |
| NaOH/HCl | q.s. pH 7.2 |
| Purified Water | q.s. 100 mL |

### Example 8

| FORMULATION FOR TOPICAL OCULAR SUSPENSION | |
|---|---|
| Ingredient | Amount (wt.%) |
| 4,9(11)-Pregnadien-17α,21-diol-3,20-dione-21-acetate | 1.0 |
| Tyloxapol | 0.01 to 0.05 |
| HPMC | 0.5 |
| Benzalkonium chloride | 0.01 |
| Sodium chloride | 0.8 |
| Edetate Disodium | 0.01 |
| NaOH/HCl | q.s. pH 7.4 |
| Purified Water | q.s. 100 mL |

The formulation is prepared by first placing a portion of the purified water into a beaker and heating to 90°C. The hydroxypropylmethylcellulose (HPMC) is then added to the heated water and mixed by means of vigorous vortex stirring until all of the HPMC is dispersed. The resulting mixture is then allowed to cool while undergoing mixing in order to hydrate the HPMC. The resulting solution is then sterilized by means of autoclaving in a vessel having a liquid inlet and a hydrophobic, sterile air vent filter.

The sodium chloride and the edetate disodium are then added to a second portion of the purified water and dissolved. The benzalkonium chloride is then added to the solution, and the pH of the solution is adjusted to 7.4 with 0.1M NaOH/HCl. The solution is then sterilized by means of filtration.

The 4,9(11)-Pregnadien-17a,21-diol-3,20-dione-21-acetate is sterilized by either dry heat or ethylene oxide. If ethylene oxide sterilization is selected, aeration for at least 72 hours at 50°C is necessary. The sterilized 4,9(11)-Pregnadien-17a,21-diol-3,20-dione-21-acetate is weighed aseptically and placed into a pressurized ballmill container. The tyloxapol, in sterilized aqueous solution form, is then added to the ballmill container. Sterilized glass balls are then added to the container and the contents of the container are milled aseptically at 225 rpm for 16 hours, or until all particles are in the range of approximately 5 microns.

Under aseptic conditions, the micronized drug suspension formed by means of the preceding step is then poured into the HPMC solution with mixing. The ballmill container and balls contained therein are then rinsed with a portion of the solution containing the sodium chloride, the edetate disodium and benzalkonium chloride. The rinse is then added aseptically to the HPMC solution. The final volume of the solution is then adjusted with purified water and, if necessary, the pH of the solution is adjusted to pH 7.4 with NaOH/HCl. The formulation will be given topically, in a therapeutically effective amount. In this instance, the phrase "therapeutically effective amount" means an amount which is sufficient to substantially prevent or reverse any ocular neovascularization. The dosage regimen used will depend on the nature of the neovascularization, as well as various other factors such as the patient's age, sex, weight, and medical history.

### Reference Example 9

| FORMULATION FOR ORAL ADMINISTRATION |
|---|
| Tablet: |
| 5-100 mg 21-Nor-5β-pregnan-3α-17α-20-triol with inactive ingredients such as starch, lactose and magnesium stearate can be formulated according to procedures known to those skilled in the art of tablet formulation. |

### Reference Example 10

| Formulation for Sterile Intraocular Injection | |
|---|---|
| each mL contains: | |
| 4,9(11)-Pregnadien-17α,21-diol-3,20-dione | 10-100 mg |
| Sodium Chloride | 7.14 mg |
| Potassium Chloride | 0.38 mg |
| Calcium chloride dihydrate | 0.154 mg |
| Magnesium chloride hexahydrate | 0.2 mg |
| Dried sodium phosphate | 0.42 mg |
| Sodium bicarbonate | 2.1 mg |
| Dextrose | 0.92 mg |
| Hydrochloric acid or sodium hydroxide to adjust pH to approximately 7.2 | |
| Water for injection | |

### Example 11

Inhibition of angiogenesis in the rabbit corneal neovascularization model:
The corneal pocket system of BenEzra (Am. J. Ophthalmol 86:455-461, 1978) was used to induce corneal neovascularization in the rabbit. A small Elvax pellet containing 0.5µg of lipopolysaccharide (LPS) was inserted into the middle of the corneal stroma and positioned 2.5 mm from the limbus. An additional Elvax pellet with or without 50µg of angiostatic steroid was placed next to the LPS implant. The eyes were examined daily and the area of neovascularization calculated. Results after 8 days of LPS implantation are shown in Figure 1. THF - tetrahydrocortisol; A = 4,9(11)-Pregnadien-17α,21-diol-3,20-dione-21-acetate; B = 4,9(11)-Pregnadien-17α,21-diol-3,20-dione. As can be seen, A & B totally inhibited corneal neovascularization, whereas THF partially inhibited the neovascular response.

### Reference Example 12

### Preparation of 5β-Pregnan-11β, 17α, 21-triol-20-one

### Tetrahydrocortisol-F-21-t-butyldiphenylsilyl ether (PS03842)

A solution of 4.75 g (17.3 mmol) of t-butyldiphenylchlorosilane in 5 mL of dry DMF was added dropwise to a stirred solution of 5.7 g (15.6 mmol) of tetrahydrocortisol-F (Steraloids No. P9050) and 2.3 g (19 mmol) of 4-dimethylaminopyridine (DMAP) in 30 mL of dry DMF, under N₂, at -25 to -30°C (maintained with CO₂ - MeCN). After a further 20 min at -30°C, the mixture was allowed to warm to 23°C overnight.

The mixture was partitioned between ether and water, and the organic solution was washed with brine, dried (MgSO₄), filtered and concentrated to give 10.7 g of a white foam.

This material was purified by flash column chromatography (400 g silica; 62.5 to 70% ether/hexane). The 3-siloxy isomer eluted first, followed by mixed fractions, followed by the title compound. The concentrated mixed fractions (4.0 g) were chromatographed on the same column with 35% ethyl acetate/hexane. The total yield of the 3-siloxy isomer was 0.42 g (5%), and of the title compound, 5.05 g (53.5%). Continued elution with 25% MeOH/EtOAc allowed recovery of unreacted tetrahydrocortisol-F.

### PS03842

NMR (200 MHz ¹H) (CDCl₃): δ 0.63 (s, 3H, Me-18); 1.11 (s, 9H, t-Bu); 1.12 (s, 3H, Me-19); 2.57 (t, J-13, 1H, H-8); 2.6 (s, 1H, OH-17); 3.63 (sept, J=2.5, 1H, H-3); 4.15 (br s, 1H, H-11); 4.37 and 4.75 (AB, J=20, 2H, H-21); 7.4 (m, 6H) and 7.7 (m, 4H) (Ph₂).
NMR (200 MHz ¹H) (DMSO-d₆): δ 0.64 (s, 3H, Me-18); 1.02 (s, 9H, t-Bu); 1.07 (s, 3H, Me-19); 2.50 (t, J=13, 1H, H-8); 3.37 (m, 1H, H-3); 3.94 (d, J=2, 1H, OH-11); 4.00 (br s, 1H, H-11); 4.42 (d, J=5, 1H OH-3); 4.38 and 4.83 (AB, J=20, 2H, H-21); 5.11 (s, 1H, OH-17); 7.45 (m, 6H) and 7.6 (m, 4H) (Ph₂).

NMR (50.3 - MHz ¹³C) (CDCl₃): 17.4 (C-18); 19.3 (C-16); 23.7 (C-15); 26.3 (C-7); 26.6 (C-19); 26.8 (Me₃C); 27.2 (C-6); 30.9 (C-2); 31.5 (C-8); 34.1 (Me₃C); 34.8 (C-10); 35.2 (C-1); 36.2 (C-4); 39.7 (C-13); 43.5 (C-5); 44.3 (C-9); 47.4 (C-12); 52.1 (C-14); 67.8 (C-11); 68.9 (C-21); 71.7 (C-3); 89.8 (C-14); 127.8, 129.8, 132.8, 132.9, 135.7, 135.8 (diastereotopic Ph₂); 208.8 (C-20). Underlined resonances showed inversion in the APT experiment. Assignments: E. Breitmaier, W. Voelter "Carbon-13 NMR Spectroscopy," 3d ed., VCH, 1987; pp. 345-348.
IR (KBr) 3460, 2930, 2860, 1720, 1428, 1136, 1113, 1070, 1039, 703 cm⁻¹.

This compound did not show a sharp melting point but turned to a foam at 80-100°C. Numerous attempts at recrystallization failed.

### 5β-Pregnan-11β, 17α, 21-triol-20-one

A solution of PS03842 (0.91 g, 1.50 mmol) and thiocarbonyl diimidazole (1.05 g, 5.9 mmol) in 8 mL of anhydrous dioxane was refluxed under N₂ for 3.5 h. The cooled solution was partitioned between ether and water and the organic solution was washed with brine, dried (MgSO₄), filtered and concentrated. The residue was chromatographed (120 g SiO₂, 35% EtOAc/hexane) giving 0.86 g (80%) of the imidazolyl thioester.

A solution of 0.75 g (1.05 mmol) of this compound in 100 mL of anhydrous dioxane was added dropwise over 2.2 h to a rapidly stirred, refluxing solution of 1.6 mL (5.9 mmol) of Bu₃SnH in 100 mL of anhydrous dioxane under N₂. After a further 1 h at reflux, the solution was cooled, concentrated and the residue chromatographed (200 g SiO₂, 9% EtOAc/hexane) giving 0.43 g (70%) of the 3-deoxy-21-silyl ether. This material was dissolved in 20 mL of methanol; Bu₄NF·3H₂O (0.50 g, 1.6 mmol) was added, and the mixture was heated to reflux under N₂ for 4 h. The cooled solution was diluted with 2 volumes of EtOAc, concentrated to 1/4 volume, partitioned (EtOAc/H₂O), and the organic solution was washed with brine, dried (MgSO₄), filtered and concentrated. The residue (0.40 g) was chromatographed (30 g SiO₂, 40% EtOAc/hexane) to give 0.25 g (98%) of an oil.

This oil was crystallized (n-BuCl) to afford 0.14 g of the title compound as a white solid, m.p. 167-170°C.
IR (KBr): 3413 (br), 2934, 1714, 1455, 1389, 1095, 1035 cm⁻¹.
MS (CI): 351 (M +1).
NMR (200 MHz ¹H, DMSO-d₆): δ 0.69 (s, 3H, Me-18); 1.14 (s, 3H, Me-19); 0.8-2.0 (m); 2.5 (t, J=13, 1H, H-8); 3.96 (d, J=2, 1H, OH-11); 4.1 (br s, 1H, H-11); 4.1 and 4.5 (AB, further split by 5 Hz, 2H, H-21); 4.6 (t, J=5, 1H, OH-21); 5.14 (s, 1H, OH-17).
Anal. Calc'd for C₂₁H₃₄O₄: C, 71.96; H, 9.78.
Found: C, 71.69; H, 9.66.

### Reference Example 13

### Preparation of 21-Methyl-5β-pregnan-3α, 11β, 17α, 21-tetrol-20-one 21-methyl ether

Sodium hydride (60% oil dispersion, 0.10 g, 2.5 mmol) was added to a stirred solution of tetrahydrocortisol-F (0.73 g, 2.0 mmol) and CH₃I (0.60 mL, 9.6 mmol) in 8 mL of anhydrous DMF under N₂. Hydrogen was evolved, and the temperature rose to 35°C. After 1 h, the mixture was diluted with EtOAc, extracted with water (until neutral) and brine, dried (MgSO₄), filtered and concentrated. The residue was chromatographed (70 g SiO₂, 80% EtOAc/hexane) to give 0.17 g of a white solid, MS (CI) = 395 (M +1). This material was recrystallized (EtOAc-n-BuCl) to afford 0.12 g (16%) of the title compound as a feathery white solid, m.p. 208-213 °C.
IR (KBr): 3530, 3452, 2939, 2868, 1696 (s, CO), 1456, 1366, 1049 cm⁻¹.
NMR (200 MHz ¹ H, DMSO-d₆): δ 0.74 (s, 3H, Me-18); 1.09 (s, 3H, Me-19); 1.14 (d, J=6.6, 3H, C-21 Me); 0.8-2.0 (m); 2.47 (t, J=13, 1H, H-8); 3.18 (s, 3H, OMe); 3.35 (m, 1H, H-3); 4.00 (d, J=2, 1H, OH-11); 4.07 (br s, 1H, H-11); 4.37 (q, J=6.6, 1H, H-21); 4.43 (d, J=5, 1H, OH-3); 5.16 (s, 1H, OH-17).
Anal. Calc'd for C₂₃H₃₈O₅: C, 70.01; H, 9.71.
Found: C, 70.06; H, 9.76.

### Reference Example 14

### Preparation of 3β-Azido-5β-pregnan-11β, 17α,21-triol-20-one-21-acetate

A solution of triphenylphosphine (2.6 g, 10 mmol) in 10 mL of toluene was carefully added to a stirred solution of PS03842 (see Example 4) (1.75 g, 2.90 mmol), diphenylphosphoryl azide (2.2 mL, 10.2 mmol) and diethyl azodicarboxylate (1.55 mL, 10 mmol) under N₂, keeping the internal temperature below 35°C (exothermic). The solution was stirred for 1.2 h, then diluted with ether, washed with water and brine, dried (MgSO₄), filtered and concentrated and the residue (9.5 g, oil) chromatographed 175 g SiO₂, 15% EtOAc/hexane) giving 1.83 g of a viscous oil.

A solution of 1.73 g of this material and 1.75 g (5.5 mmol) of Bu₄NF·3H₂O in 20 mL of methanol was refluxed under N₂ for 2.5 h. The crude product (1.94 g) was isolated with ethyl acetate and chromatographed (100 g SiO₂, 50% EtOAc/hexane) giving 0.60 g (56%) of a white semisolid. Trituration (4:1 hexane-ether) gave 0.57 g (53%) of a solid.

A stirred solution of 0.40 g of this material in 3 mL of dry pyridine was treated with 0.3 mL of acetic anhydride and stirred overnight at 23°C under N₂. The mixture was quenched with 1 mL of methanol, stirred for 15 min, diluted with ether, washed with 1 M aqueous HCl, water (until neutral), brine, dried (MgSO4), filtered and concentrated. The residue (0.41 g, oil) was chromatographed (35 g SiO₂, 33% EtOAc/hexane) to afford 0.33 g (76%) of the title compound as a white foam, m.p. 80-90°C (dec).
IR (KBr): 3505, 2927, 2866, 2103 (vs), 1721 (sh 1730), 1268, 1235 cm⁻¹.
NMR (200 MHz ¹H, CDCl₃): δ 0.92 (s, 3H, Me-18); 1.21 (s, 3H, Me-19); 1.0-2.1 (m); 2.17 (s, 3H, Ac); 2.25 (s 1H, OH-17); 2.74 (m, 1H, H-8); 3.97 (br s, 1H, H-3); 4.31 (br s, 1H, H-11); 4.94 (AB, J=17, Δν=60, 2H, H-21).
Anal. Calc'd for C₂₃H₃₅N₃O₅: C, 63.72; H, 8.14; N, 9.69.
Found: C, 63.39; H, 8.18; N, 9.45.

### Reference Example 15

### Preparation of 3β-Acetamido-5β-pregnan-11β, 17α-21-triol-20-one-21-acetate

A solution of 3β-azido-5β-pregnan-11β,17α,21-triol-20-one-21-acetate (0.15 g, 0.35 mmol) in 8 mL of absolute ethanol containing 0.03 g of 10% Pd on C was stirred under H₂ ( 1 atm) at 23°C for 2 h. The mixture was filtered and concentrated, the residue dissolved in EtOAc, the basic material extracted into 1 M aqueous HCl, liberated (Na₂CO₃), extracted (EtOAc) and the organic extract washed with water (until neutral) and brine, dried (MgSO₄), filtered and concentrated to provide 58 mg of a solid.

This material was acetylated (1.0 mL of dry pyridine, 0.20 mL of Ac₂O, 23°C, N₂, overnight), followed by workup (as described for the steroid of Example 14 [last step]) affording a crude product that was chromatographed (25 g SiO₂, EtOAc). This product was triturated with ether to afford 51 mg (33%) of product as a white solid, m.p. 179-181°C.
MS (CI, isobutane): (M +1) = 450 (M⁺), 432, 391, 371, 348.
IR (KBr): 3398 (br), 2932, 2865, 1720 (sh. 1740), 1652, 1538, 1375, 1265, 1236 cm⁻¹.
NMR (200 MHz ¹H, CDCl₃): δ 0.89, 1.22, 1.99, 2.17 (all s, 3H); 1.0-2.2 (m); 2.7 (t, J=13, 1H, H-8); 3.03 (s, 1H, OH-17); 4.2 (br s, 1H, H-11); 4.3 (br s, 1H, H-3); 4.96 (AB, J=17.5, Δν=42, 2H, H-21); 5.8 (d, J=10, 1H, NH).

## Claims

1. Use of 4,9(11)-pregnadien-17α,21-diol-3,20-dione-21-acetate for the preparation of a medicament for preventing and/or treating neovascularization.

2. Use according to claim 1, wherein the medicament is for preventing and/or treating ocular neovascularization.

3. Use according to claim 2, wherein the medicament is for preventing and/or treating retinal diseases including diabetic retinopathy, chronic glaucoma, retinal detachment, sickle cell retinopathy and senile macular degeneration due to subretinal neovascularization; rubeosis iritis; inflammatory diseases; chronic uveitis; neoplasms including retinoblastoma and pseudoglioma; Fuch's heterochromic iridocyclitis; neovascular glaucoma; corneal neovascularization including inflammatory, transplantation and developmental hypoplasia of the iris; neovascularization resulting following a combined vitrectomy and lensectomy; vascular diseases including retinal ischemia, choroidal vascular insufficiency, choroidal thrombosis and carotid artery ischemia; pterigium; neovascularization of the optic nerve; and neovascularization due to penetration of the eye or contusive ocular injury.

## Patentansprüche

1. Verwendung von 4,9(11)-Pregnadien-17α,21-diol-3,20-dion-21-acetat zur Herstellung eines Arzneimittels zur Verhütung und/oder Behandlung von Gefäßneubildung.

2. Verwendung nach Anspruch 1, wobei das Arzneimittel zur Verhütung oder Behandlung einer Gefäßneubildung im Auge dient.

3. Verwendung nach Anspruch 2, wobei das Arzneimittel zur Verhütung und/oder Behandlung von Netzhautkrankheiten einschließlich diabetischer Retinopathie, chronischem Glaukom, Netzhautablösung, Sichelzellen-Retinopathie und seniler Makulaatrophie auf Grund einer subretinalen Gefäßneubildung; Rubeose; entzündlichen Krankheiten; chronischer Uveitis; Neoplasmen einschließlich Retinoblastom und Pseudogliom; Fuchs' Heterochromie mit Iridocyclitis; neovaskulärem Glaukom; Hornhautgefäßneubildung einschließlich entzündlicher, Transplantations- und Entwicklungshypoplasie der Iris; Gefäßneubildung entstehend nach kombinierter Vitrectomie und Linsectomie; Gefäßkrankheiten einschließlich Netzhautischämie, Choroid-Gefäßinsuffizienz, Choroid-Thrombose und Ischämie der Arteria carotis; Pterigium; Gefäßneubildung des Nervus opticus; und Gefäßneubildung auf Grund einer Penetration im Auge oder einer stumpfen Augenverletzung.

## Revendications

1. Utilisation de 4,9(11)-pregnadiène-17α,21-diol-3,20-dione-21-acétate pour la préparation d'un médicament pour prévenir et/ou traiter une néovascularisation.

2. Utilisation suivant la revendication 1, dans laquelle le médicament est utilisé pour prévenir et/ou traiter une néovascularisation oculaire.

3. Utilisation suivant la revendication 2, dans laquelle le médicament est utilisé pour prévenir et/ou traiter des maladies rétiniennes et notamment la rétinopathie diabétique, le glaucome chronique, le détachement rétinien, la rétinopathie à drépanocytes et la dégénération maculaire sénile due à une néovascularisation subrétinienne, les rougeurs irritantes, les maladies inflammatoires, l'uvéite chronique, les néoplasmes et notamment le rétinoblastome et le pseudogliome, l'iridocyclite hétérochromique de Fuch, le glaucome néovasculaire, la néovascularisation cornéenne et notamment inflammatoire, de transplantation et l'hypoplasie de développement de l'iris, la néovascularisation résultant de la suite d'une vitrectomie et d'une ablation du cristallin combinées, les maladies vasculaires et notamment l'ischémie rétinienne, l'insuffisance vasculaire choroïdienne, la thrombose choroïdienne et l'ischémie de l'artère carotide, le ptérygion, la néovascularisation du nerf optique et la néovascularisation due à une pénétration de l'oeil ou une lésion oculaire contusionnelle.
